# FASCICULE DE BREVET EUROPEEN

(11) **EP 4 565 377 B1**
(45) Date de publication et mention de la délivrance du brevet: **22.07.2026**
(21) Numéro de dépôt: 23761560.4
(22) Date de dépôt: 02.08.2023
(51) Int. Cl.: B05B 11/10, A61M 11/00, A61M 15/00, A61M 15/08

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 03.08.2022 FR 2208054
(43) Date de publication de la demande: 11.06.2025
(73) Titulaire: APTAR France SAS, 27110 Le Neubourg (FR)
(72) Inventeur: PETIT, Ludovic, 27110 VITOT (FR)
(74) Mandataire: CAPRI
(86) Numéro de dépôt international: PCT/FR2023/051230
(87) Numéro de publication internationale: WO 2024/028556

(56) Documents cités:
- EP-A1- 3 120 934
- EP-A2- 1 136 135
- WO-A1-2020/128269
- CN-U- 216 187 535
- FR-A1- 2 343 137
- FR-A3- 2 563 287
- JP-B2- 5 477 865

## Description

La présente invention concerne un dispositif de distribution de produit fluide.

Les documents FR2343137 et FR2403465 décrivent un dispositif comportant une pompe comprenant un corps de pompe dans lequel peut se déplacer un piston, monté coulissant sur une tige d'actionnement, l'effort de poussée exercé sur la tige par l'utilisateur étant transmis au piston par l'intermédiaire d'un ressort, de façon qu'en actionnant la tige d'actionnement, il se produise un déplacement relatif du piston par rapport à la tige.

Le document WO2020128269A1 décrit une version améliorée de la pompe ci-dessus. Le document CN216187535U décrit un autre exemple de pompe de l'art antérieur.

Ces pompes présentent toutefois certains inconvénients. Ainsi, elles comportent deux ressorts métalliques, ce qui n'est pas avantageux d'un point de vue du recyclage. De plus, elles comportent un fouloir, coopérant avec le ressort de rappel et nécessaire pour réaliser l'amorçage de la pompe.

La présente invention a pour but de fournir un dispositif de distribution de produit fluide qui ne reproduit pas les inconvénients susmentionnés.

La présente invention a aussi pour but de fournir un dispositif de distribution de produit fluide qui améliore le recyclage en supprimant les ressorts métalliques.

La présente invention a aussi pour but de fournir un dispositif de distribution de produit fluide qui simplifie l'amorçage.

La présente invention a également pour but de fournir un dispositif de distribution de produit fluide qui soit simple et peu coûteux à fabriquer et à assembler.

La présente a donc pour objet un dispositif de distribution de produit fluide comportant une tête de distribution pourvue d'un orifice de distribution, ladite tête de distribution étant assemblée sur une pompe comportant:
- un corps de pompe, comportant une chambre de pompe, ledit corps de pompe comportant une ouverture supérieure fermée par un piston et une ouverture inférieure obturée par un clapet d'entrée, maintenu en place par un porte-clapet fixé dans ledit corps de pompe,
- ledit piston coulissant de manière étanche dans ledit corps de pompe autour d'une tige axiale creuse reliée à ladite tête de distribution, ladite tige axiale creuse étant pourvue d'un canal axial interne fermé axialement du côté inférieur et communiquant avec l'extérieur par un orifice radial, ledit piston comportant sur un bord radialement externe une lèvre d'étanchéité externe pour assurer l'étanchéité contre ledit corps de pompe, et sur un bord radialement interne une lèvre d'étanchéité interne, pour obturer de manière étanche au repos ledit orifice radial,
- un organe de rappel élastique pour solliciter ladite pompe vers sa position de repos, et la ramener vers cette position de repos après chaque actionnement,
ledit porte-clapet comportant un manchon axial se projetant axialement vers le haut, dont un bord supérieur coopère par butée mécanique avec ladite lèvre d'étanchéité interne dudit piston, lors de l'amorçage, quand ladite chambre de pompe contient de l'air, pour déplacer ledit piston axialement vers le haut par rapport à ladite tige pour ouvrir ledit orifice radial et permettre l'expulsion de l'air contenu dans ladite chambre de pompe.

Avantageusement, ledit organe de rappel élastique est non métallique.

Avantageusement, ledit organe de rappel élastique est un soufflet disposé entre ladite tête de distribution et une partie fixée sur ledit corps de pompe, tel qu'une bague de fixation.

Avantageusement, un insert axial creux est fixé dans ladite tige axiale creuse, ledit insert axial creux comportant ledit orifice radial.

Avantageusement, une bague de fixation est prévue pour fixer ledit corps de pompe sur un réservoir avec interposition d'un joint de col, un joint de bague étant prévu pour assurer l'étanchéité de ladite bague de fixation avec ledit corps de pompe.

Avantageusement, en position de repos, un bord axial supérieur dudit piston coopère de manière étanche avec ledit joint de bague.

Avantageusement, un évent est prévu dans ledit corps de pompe pour permettre le remplacement par de l'air du produit expulsé par la pompe à chaque actionnement.

Avantageusement, ledit piston comporte au moins une lèvre déformable coopérant lors de l'actionnement avec une partie tronconique de ladite tige axiale creuse pour générer une précompression.

Avantageusement, ledit bord supérieur dudit manchon axial comporte au moins une ouverture, pour laisser passer l'air à l'amorçage.

Avantageusement, chaque ouverture est formée par une encoche réalisée dans ledit bord supérieur dudit manchon.

Ces caractéristiques et avantages et d'autres apparaîtront plus clairement au cours de la description détaillée suivante, faite en référence aux dessins joints, donnés à titre d'exemples non limitatifs, et sur lesquels:
la figure 1 est une vue schématique en section transversale d'un dispositif de distribution de produit fluide comportant une pompe de distribution de produit fluide selon un premier mode de réalisation avantageux de l'invention, en position de repos,
les figures 2 et 3 sont des vues schématiques de détail en section transversale montrant la précompression, respectivement avant et en cours d'actionnement, du dispositif de la figure 1,
la figure 4 est une vue schématique de détail en section transversale d'un dispositif de distribution de produit fluide comportant une pompe de distribution de produit fluide selon un second mode de réalisation avantageux de l'invention, en position d'actionnement lors de l'amorçage, et
la figure 5 est une vue schématique de détail en perspective du porte-clapet du dispositif de la figure 4.

Dans la description, les termes "supérieur", "inférieur", "haut" et "bas" se réfèrent à la position droite du dispositif représentée sur les figures. Les termes "axial" et "radial" se réfèrent à l'axe central vertical de la pompe.

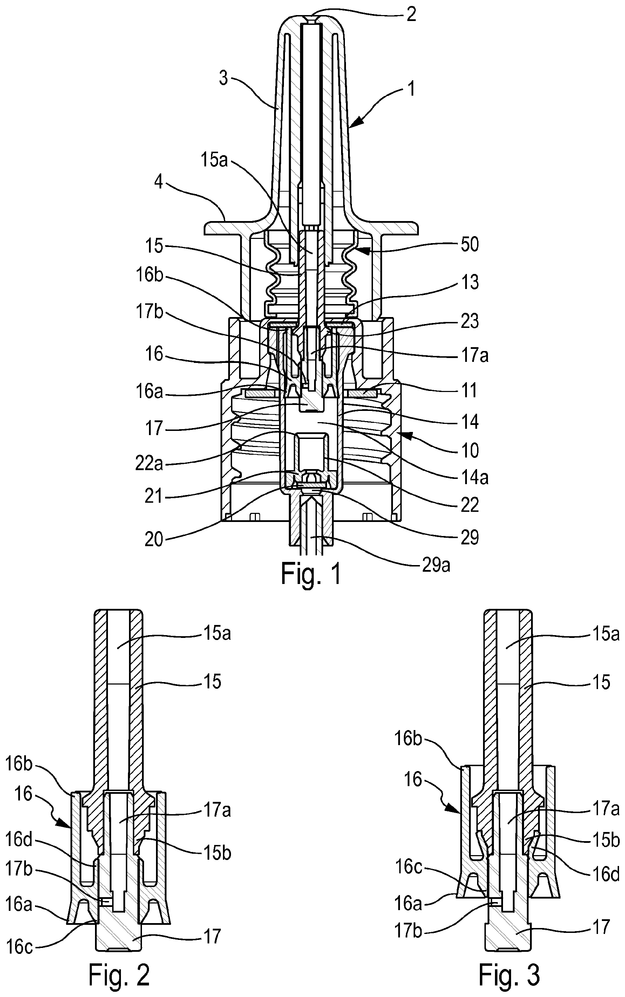

La figure 1 représente une pompe selon un mode de réalisation avantageux de l'invention, qui améliore une pompe de l'art antérieur telle que décrite dans le document WO2020128269A1.

Cette pompe comprend un corps de pompe 14 contenant une chambre de pompe 14a. Le corps de pompe 14 est ouvert axialement des deux côtés, avec une ouverture supérieure fermée par un piston 16 et une ouverture inférieure 29 obturée par un clapet d'entrée 20.

Une bague de fixation 10 est prévue pour fixer le corps de pompe 14 sur le col d'un réservoir (non représenté) avec interposition d'un joint de col 11. Un joint de bague 13 assure l'étanchéité de la bague de fixation 10 avec le corps de pompe 14.

Le piston 16 coulisse de manière étanche dans le corps de pompe 14, autour d'une tige axiale 15 creuse avec un premier canal axial interne 15a.

Le premier canal axial interne 15a débouche axialement vers le haut de la tige 15 pour se connecter à une tête de distribution 1 pourvue d'un orifice de distribution 2.

La tête de distribution 1 comporte un embout nasal 3 destiné à être au moins partiellement inséré dans une narine lors de l'utilisation, ainsi qu'une bride radiale 4 formant appui pour les doigts de l'utilisateur lors de l'actionnement.

Vers le bas, le premier canal axial interne 15a est également ouvert axialement et la tige axiale creuse 15 reçoit un insert axial creux 17.

Cet insert axial creux 17 comporte un second canal axial interne 17a qui débouche axialement vers le haut dans le premier canal axial interne 15a de la tige axiale creuse 15.

Vers le bas, le second canal axial interne 17a est fermé axialement, et l'insert axial creux comporte un orifice radial 17b pour relier le second canal axial interne 17a avec l'extérieur de l'insert axial creux 17.

Eventuellement, la tige 15 et l'insert 17 pourraient être formés d'une seule pièce monobloc, par exemple par moulage.

Un organe de rappel élastique 50, non métallique, est prévu pour solliciter la pompe vers sa position de repos, et la ramener vers cette position de repos après chaque actionnement.

Dans l'exemple représenté, cet organe de rappel élastique 50 est formé par un soufflet disposé entre la tête de distribution 1 et la bague de fixation 10, qui sollicite élastiquement la tête de distribution vers sa position de repos. Lors de l'actionnement, ce soufflet 50 se comprime, de sorte qu'après l'actionnement, le soufflet comprimé va élastiquement revenir vers sa position non comprimée, entrainant avec lui la tête de distribution 1 vers sa position de repos, ce qui à son tour ramène le piston 16 vers sa position de repos en aspirant la prochaine dose de produit fluide dans la chambre de pompe 14a.

Un bord radialement externe du piston 16 forme une lèvre d'étanchéité externe 16a, pour assurer l'étanchéité contre la surface interne du corps de pompe 14.

Un bord axial supérieur 16b du piston 16 assure au repos l'étanchéité contre le joint de bague 13.

Le corps de pompe 14 est obturé vers le bas par le clapet d'entrée 20, maintenu en place par un porte-clapet 21. Ce clapet d'entrée 20 obture l'ouverture inférieure 29, communiquant avec l'intérieur du réservoir, et sur laquelle on peut connecter un tube plongeur 29a, de la manière classique bien connue.

La tige 15 comporte un épaulement 23, qui sert à limiter le déplacement vers le haut de la tige 15, en formant une butée avec le joint de bague 13.

Quand la pompe est au repos, le bord supérieur 16b du piston 16 est appliqué contre le joint de bague 13.

L'étanchéité entre le piston 16 et l'insert axial creux 17 est assurée par une partie interne du piston 16, de préférence de forme conique, formant lèvre d'étanchéité interne 16c.

Au repos, l'orifice radial 17b est au droit de la lèvre d'étanchéité interne 16c, de façon à être parfaitement obturé. L'étanchéité entre l'extérieur et l'intérieur du réservoir est donc assurée d'une part par le contact entre le bord supérieur 16b du piston 16 et le joint de bague 13, et d'autre part entre la lèvre d'étanchéité interne 16c et l'insert axial creux 17.

Pour réaliser une précompression lors de l'actionnement, qui permet notamment d'assurer une bonne distribution de la totalité de la dose sans risque de course d'actionnement partielle, le piston 16 peut comporter au moins une lèvre déformable 16d coopérant avec une partie tronconique 15b de la tige axiale creuse 15. Les figures 2 et 3 illustrent cette précompression. En variante, la partie tronconique pourrait aussi être réalisée sur l'insert axial creux 17.

Au repos, la lèvre 16d est sensiblement non déformée, et au début de la course d'actionnement, elle va coopérer avec la partie tronconique 15b qui va progressivement déformer ladite lèvre 16d. Cette déformation élastique génère une résistance à l'actionnement, et donc une précompression, qui requiert une certaine force seuil pour réaliser l'actionnement de la pompe.

De plus, cette déformation élastique de la lèvre 16d sollicite élastiquement le piston 16 vers sa position de repos, dans laquelle la lèvre d'étanchéité interne 16c du piston obture l'orifice radial 17b de l'insert axial creux 17. Ainsi, après chaque actionnement, dès que l'utilisateur relâche sa pression sur la tête de distribution 1, le piston 16 revient automatiquement vers sa position d'obturation de l'orifice radial 17b, permettant ainsi un chargement de la chambre de pompe 14a avec une nouvelle dose de produit fluide aspirée depuis le réservoir.

Un évent (non représenté) peut avantageusement être prévu dans le corps de pompe 14 pour permettre le remplacement par de l'air du produit expulsé par la pompe à chaque actionnement.

La pompe décrite ci-dessus fonctionne de la façon suivante. Au repos, l'étanchéité à l'intérieur du réservoir (non représenté) est assurée par le joint de col 11 entre la bague 10 et le réservoir (non représenté), par le joint de bague 13 entre la bague 10 et le corps de pompe 14, par le contact entre le bord supérieur 16b du piston 16 et le joint de bague 13, et par la lèvre d'étanchéité interne 16c du piston 16 contre l'insert axial creux 17.

Le réservoir, non représenté sur les dessins, et qui peut être quelconque, est rempli d'un liquide, en particulier pharmaceutique, à distribuer, notamment à pulvériser.

La bague de fixation 10 peut être en matière plastique. Elle peut être vissée, sertie, encliquetée ou fixée autrement sur le réservoir.

L'utilisateur doit d'abord amorcer la pompe en la faisant fonctionner pour chasser l'air jusqu'à ce que la chambre de pompe 14a soit remplie de liquide. Lors de l'amorçage, l'air contenu dans la chambre de pompe 14a se comprime, et c'est par une butée mécanique entre le porte-clapet 21 et le piston 16 que l'insert 17 et la tige 15 peuvent se déplacer par rapport au piston 16 pour ouvrir l'orifice radial 17b et permettre l'expulsion de l'air comprimé. Lorsque la pompe revient en position de repos, du liquide est aspiré dans la chambre de pompe 14a à travers le tube plongeur 29a, de manière bien connue.

Selon l'invention, le porte-clapet 21 comporte un manchon axial 22 se projetant axialement vers le haut, dont le bord supérieur 22a coopère lors de l'amorçage avec la lèvre d'étanchéité interne 16c pour déplacer le piston 16 axialement vers le haut, pour ouvrir l'orifice radial 17b et permettre l'expulsion de l'air contenu dans la chambre de pompe 14a.

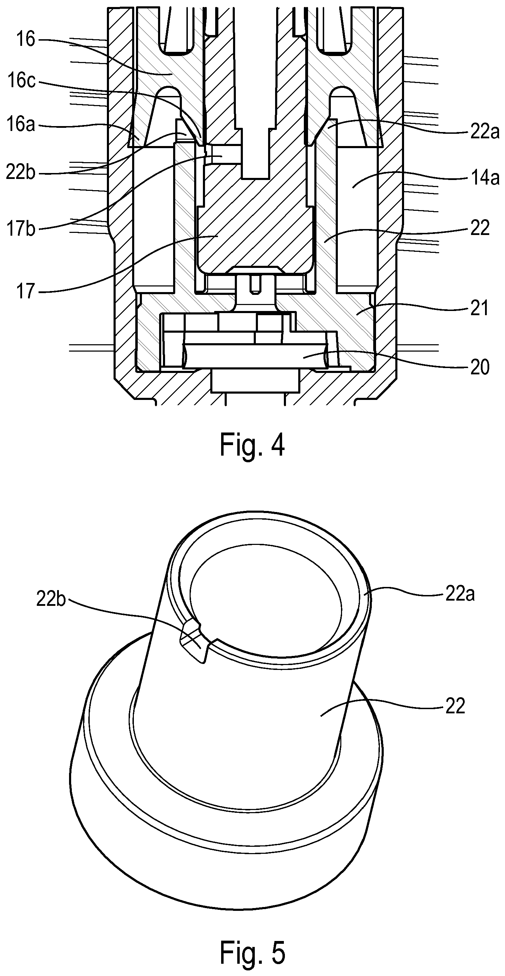

Avantageusement, comme représenté dans le second mode de réalisation des figures 4 et 5, le bord supérieur 22a du manchon axial 22 peut comporter une ou plusieurs ouvertures 22b, pour laisser passer l'air à l'amorçage. Ainsi, comme visible sur la figure 4, l'évacuation de l'air est assurée même en cas de contact sur toute la périphérie entre le bord supérieur 22a du manchon et la lèvre d'étanchéité interne 16c du piston.Avantageusement, chaque ouverture 22b peut être formée par une encoche réalisée dans le bord supérieur 22a du manchon 22.

La présente invention permet donc de réaliser un amorçage fiable sans utiliser de pièce supplémentaire, tel qu'un fouloir, intercalé entre le piston 16 et la tige 15 et/ou l'insert 17, comme c'est le cas dans la pompe du document WO2020128269A1.

Après amorçage, la pompe est prête à être utilisée pour distribuer une dose de liquide à chaque actionnement.

Pour réaliser un tel actionnement, l'utilisateur appuie axialement sur la tête de distribution 1 pour enfoncer la tige 15. Le liquide étant incompressible, le piston 16 se déplace autour de l'insert 17 en déformant la lèvre déformable 16d sur le profil tronconique 15b de la tige 15. Quand la pression est suffisante pour surmonter la résistance à la déformation de la lèvre déformable 16d, le piston 16 découvre l'orifice radial 17b, permettant l'expulsion du liquide hors de la chambre de pompe 14a.

Le liquide est expulsé rapidement par l'orifice radial 17b, le second canal interne 17a et le premier canal interne 15a, vers la tête de distribution 1 assemblée sur la pompe.

Après quoi, l'utilisateur cesse d'exercer une pression sur la tige 15. La lèvre déformable 16d sollicite immédiatement le piston 16 à revenir vers sa position de repos, ce qui assure l'obturation de l'orifice radial 17b, et ferme la chambre de pompe 14a par rapport à la tête de distribution 1. Parallèlement, le soufflet 50 ramène la tête de distribution 1 dans sa position de repos, ce qui entraine vers le haut la tige 15 et l'insert 17. Ceci crée une dépression dans la chambre de pompe 14a, de sorte que du liquide est aspiré par l'orifice 29, en soulevant le clapet 20, tandis que de l'air remplace ce liquide dans le réservoir, en passant par l'ouverture centrale du joint de bague 13 autour de la tige 15, et par l'évent 35. Quand le bord supérieur 16b du piston 16 vient en contact du joint de bague 13, l'air ne peut plus rentrer, et le piston 16 est bloqué, la pompe a repris sa position de repos et est prête pour un nouvel actionnement.

Un avantage de la pompe représentée sur la figure 1 est qu'elle ne comporte aucune pièce métallique, ce qui d'une part rend la fabrication et l'assemblage de la pompe moins complexe et donc moins coûteux, et ce qui d'autre part est avantageux pour le recyclage.

Un autre avantage est que la pompe assure un amorçage fiable sans pièce intermédiaire entre le piston et la tige et/ou l'insert.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant une tête de distribution (1) pourvue d'un orifice de distribution (2), ladite tête de distribution étant assemblée sur une pompe comportant:
- un corps de pompe (14), comportant une chambre de pompe (14a), ledit corps de pompe (14) comportant une ouverture supérieure fermée par un piston (16) et une ouverture inférieure (29) obturée par un clapet d'entrée (20), maintenu en place par un porte-clapet (21) fixé dans ledit corps de pompe (14),
- ledit piston (16) coulissant de manière étanche dans ledit corps de pompe (14) autour d'une tige axiale creuse (15) reliée à ladite tête de distribution (1), ladite tige axiale creuse (15) étant pourvue d'un canal axial interne (15a) fermé axialement du côté inférieur et communiquant avec l'extérieur par un orifice radial (17b), ledit piston (16) comportant sur un bord radialement externe une lèvre d'étanchéité externe (16a) pour assurer l'étanchéité contre ledit corps de pompe (14), et sur un bord radialement interne une lèvre d'étanchéité interne (16c), pour obturer de manière étanche au repos ledit orifice radial (17b),
- un organe de rappel élastique (50) pour solliciter ladite pompe vers sa position de repos, et la ramener vers cette position de repos après chaque actionnement,
**caractérisé en ce que** ledit porte-clapet (21) comporte un manchon axial (22) se projetant axialement vers le haut, dont un bord supérieur (22a) coopère par butée mécanique avec ladite lèvre d'étanchéité interne (16c) dudit piston (16), lors de l'amorçage, quand ladite chambre de pompe (14a) contient de l'air, pour déplacer ledit piston (16) axialement vers le haut par rapport à ladite tige axiale creuse (15) pour ouvrir ledit orifice radial (17b) et permettre l'expulsion de l'air contenu dans ladite chambre de pompe (14a).

2. Dispositif selon la revendication 1, dans lequel ledit organe de rappel élastique (50) est non métallique.

3. Dispositif selon la revendication 2, dans lequel ledit organe de rappel élastique (50) est un soufflet disposé entre ladite tête de distribution (1) et une partie fixée sur ledit corps de pompe (14), tel qu'une bague de fixation (10).

4. Dispositif selon l'une quelconque des revendications précédentes, dans lequel un insert axial creux (17) est fixé dans ladite tige axiale creuse (15), ledit insert axial creux (17) comportant ledit orifice radial (17b).

5. Dispositif selon l'une quelconque des revendications précédentes, dans lequel une bague de fixation (10) est prévue pour fixer ledit corps de pompe (14) sur un réservoir avec interposition d'un joint de col (11), un joint de bague (13) étant prévu pour assurer l'étanchéité de ladite bague de fixation (10) avec ledit corps de pompe (14).

6. Dispositif selon la revendication 5, dans lequel, en position de repos, un bord axial supérieur (16b) dudit piston (16) coopère de manière étanche avec ledit joint de bague (13).

7. Dispositif selon l'une quelconque des revendications précédentes, dans lequel un évent est prévu dans ledit corps de pompe (14) pour permettre le remplacement par de l'air du produit expulsé par la pompe à chaque actionnement.

8. Dispositif selon l'une quelconque des revendications précédentes, dans lequel ledit piston (16) comporte au moins une lèvre déformable (16d) coopérant lors de l'actionnement avec une partie tronconique (15b) de ladite tige axiale creuse (15) pour générer une précompression.

9. Dispositif selon l'une quelconque des revendications précédentes, dans lequel ledit bord supérieur (22a) dudit manchon axial (22) comporte au moins une ouverture (22b), pour laisser passer l'air à l'amorçage.

10. Dispositif selon la revendication 9, dans lequel chaque ouverture (22b) est formée par une encoche réalisée dans ledit bord supérieur (22a) dudit manchon axial (22).

## Patentansprüche

1. Vorrichtung zur Abgabe eines Fluidprodukts, die einen Abgabekopf (1) aufweist, der mit einer Abgabeöffnung (2) versehen ist, wobei der Abgabekopf auf einer Pumpe montiert ist, die aufweist:
- einen Pumpenkörper (14), der eine Pumpenkammer (14a) aufweist, wobei der Pumpenkörper (14) eine obere Öffnung, die durch einen Kolben (16) verschlossen ist, und eine untere Öffnung (29) aufweist, die durch ein Einlassventil (20) verschlossen ist, das durch einen Ventilhalter (21), der in dem Pumpenkörper (14) befestigt ist, an Ort und Stelle gehalten wird,
- wobei der Kolben (16) in dem Pumpenkörper (14) um einen hohlen axialen Schaft (15) dicht gleitet, der mit dem Abgabekopf (1) verbunden ist, wobei der hohle axiale Schaft (15) mit einem inneren axialen Kanal (15a) versehen ist, der axial auf der unteren Seite geschlossen ist und mit der Außenseite über eine radiale Öffnung (17b) kommuniziert, wobei der Kolben (16) an einem radial äußeren Rand eine äußere Dichtlippe (16a), um die Dichtigkeit gegenüber dem Pumpenkörper (14) sicherzustellen, und an einem radial inneren Rand eine innere Dichtlippe (16c) aufweist, um die radiale Öffnung (17b) in Ruhe dicht zu verschließen,
- ein elastisches Rückstellelement (50), um die Pumpe in ihre Ruhestellung zu beaufschlagen und sie nach jeder Betätigung in diese Ruhestellung zurückzubringen,
**dadurch gekennzeichnet, dass**
der Ventilhalter (21) eine axiale Hülse (22) aufweist, die axial nach oben vorspringt, deren oberer Rand (22a) beim Ansaugen, wenn die Pumpenkammer (14a) Luft enthält, durch mechanischen Anschlag mit der inneren Dichtlippe (16c) des Kolbens (16) zusammenwirkt, um den Kolben (16) bezüglich des hohlen axialen Schafts (15) axial nach oben zu verschieben, um die radiale Öffnung (17b) zu öffnen und das Ausstoßen der in der Pumpenkammer (14a) enthaltenen Luft zu ermöglichen.

2. Vorrichtung nach Anspruch 1, wobei das elastische Rückstellelement (50) nichtmetallisch ist.

3. Vorrichtung nach Anspruch 2, wobei das elastische Rückstellelement (50) ein Faltenbalg ist, der zwischen dem Abgabekopf (1) und einem Teil, der an dem Pumpenkörper (14) befestigt ist, wie etwa einem Befestigungsring (10), angeordnet ist.

4. Vorrichtung nach einem der vorhergehenden Ansprüche, wobei ein hohler axialer Einsatz (17) in dem hohlen axialen Schaft (15) befestigt ist, wobei der hohle axiale Einsatz (17) die radiale Öffnung (17b) aufweist.

5. Vorrichtung nach einem der vorhergehenden Ansprüche, wobei ein Befestigungsring (10) vorgesehen ist, um den Pumpenkörper (14) an einem Behälter unter Zwischenschaltung einer Halsdichtung (11) zu befestigen, wobei eine Ringdichtung (13) vorgesehen ist, um die Dichtigkeit des Befestigungsrings (10) mit dem Pumpenkörper (14) sicherzustellen.

6. Vorrichtung nach Anspruch 5, wobei in Ruhestellung ein oberer axialer Rand (16b) des Kolbens (16) dicht mit der Ringdichtung (13) zusammenwirkt.

7. Vorrichtung nach einem der vorhergehenden Ansprüche, wobei eine Entlüftung in dem Pumpenkörper (14) vorgesehen ist, um das Ersetzen des durch die Pumpe bei jeder Betätigung ausgestoßenen Produkts durch Luft zu ermöglichen.

8. Vorrichtung nach einem der vorhergehenden Ansprüche, wobei der Kolben (16) mindestens eine verformbare Lippe (16d) aufweist, die bei der Betätigung mit einem kegelstumpfförmigen Teil (15b) des hohlen axialen Schafts (15) zusammenwirkt, um eine Vorkompression zu erzeugen.

9. Vorrichtung nach einem der vorhergehenden Ansprüche, wobei der obere Rand (22a) der axialen Hülse (22) mindestens eine Öffnung (22b) aufweist, um die Luft beim Ansaugen durchzulassen.

10. Vorrichtung nach Anspruch 9, wobei jede Öffnung (22b) durch eine Kerbe gebildet ist, die in dem oberen Rand (22a) der axialen Hülse (22) ausgeführt ist.

## Claims

1. Device for dispensing a fluid product comprising a dispensing head (1) provided with a dispensing hole (2), said dispensing head being assembled on a pump comprising:
- a pump body (14), having a pump chamber (14a), said pump body (14) having an upper opening closed by a piston (16) and a lower opening (29) closed by an inlet valve (20), held in place by a valve holder (21) fixed in said pump body (14),
- said piston (16) sealingly sliding in said pump body (14) around a hollow axial rod (15) connected to said dispensing head (1), said hollow axial rod (15) being provided with an inner axial channel (15a) axially closed on the lower side and communicating with the outside by a radial hole (17b), said piston (16) having, on an outer radial edge, an outer sealing lip (16a) configured to seal against said pump body (14), and on an inner radial edge, an inner sealing lip (16c), configured to seal the radial hole when it is in the non-operating state (17b),
- an elastic return member (50) to bias said pump towards its non-operating position, and to guide it back to this non-operating position after each actuation,
**characterised in that** said valve holder (21) comprises an axial sleeve (22) projecting axially upwards, an upper edge (22a) of which mechanically abuts with said inner sealing lip (16c) of said piston (16), during start-up, when said pump chamber (14a) contains air, in order to move said piston (16) axially upwards with respect to said hollow axial rod (15) in order to open said radial hole(17b) and discharge the air contained in said pump chamber (14a).

2. Device according to claim 1, wherein said dispensing member (50) is non-metallic.

3. Device according to claim 2, wherein said resilient return member (50) is a bellows disposed between said dispensing head (1) and a part fixed on said pump body (14), such as a fixing ring (10).

4. Device according to any one of the preceding claims, wherein a hollow axial insert (17) is fixed in said hollow axial rod (15), said hollow axial insert (17) having said radial hole (17b).

5. Device according to any one of the preceding claims, wherein a fixing ring (10) is provided to fix said pump body (14) on a reservoir with a neck seal (11) interposed therebetween, a ring seal (13) being provided for ensuring the sealing of said fixing ring (10) with said pump body (14).

6. Device according to claim 5, wherein, in the non-operating position, an upper axial edge (16b) of said piston (16) sealingly cooperates with said ring seal (13).

7. Device according to any one the preceding claims, wherein a vent is provided in said pump body (14) to allow the product expelled by the pump to be replaced by air on each actuation.

8. Device according to any one of the preceding claims, wherein said piston (16) comprises at least one deformable lip (16d) cooperating during actuation with a frustoconical part (15b) of said hollow axial rod (15) to generate a precompression.

9. Device according to any preceding claim, wherein said upper edge (22a) of said axial sleeve (22) includes at least one opening (22b) to enable the passage of air upon start-up.

10. Device according to claim 9, wherein each opening (22b) is formed by a notch made in said upper edge (22a) of said sleeve axial (22).
